# EUROPEAN PATENT SPECIFICATION

(11) **EP 1 844 813 B1**
(45) Date of publication and mention of the grant of the patent: **14.10.2009**
(21) Application number: 06712873.6
(22) Date of filing: 02.02.2006
(51) Int. Cl.: A61N 1/30, A61N 1/04

(54) **IONTOPHORESIS APPARATUS**
IONTOPHORESEGERÄT
APPAREIL DE IONTOPHORESE

(30) Priority: 03.02.2005 JP 2005027748; 02.08.2005 US 195364
(43) Date of publication of application: 17.10.2007
(73) Proprietor: TTI ellebeau, Inc., Tokyo 150-0022 (JP)
(72) Inventor: TANIOKA, Akihiko, Tokyo 1450061 (JP); MINAGAWA, Mie, Tokyo 1080074 (JP); KANAMURA, Kiyoshi, Tokyo 140-0002 (JP); MATSUMURA, Akihiko, Tokyo 140-0002 (JP); NAKAYAMA, Mizuo, Tokyo 140-0002 (JP); MATSUMURA, Takehiko, Tokyo 140-0002 (JP); AKIYAMA, Hidero, Tokyo 140-0002 (JP)
(74) Representative: Schäfer, Matthias W.
(86) International application number: PCT/JP2006/301730
(87) International publication number: WO 2006/082873

(56) References cited:
- WO-A1-00/61220
- JP-A- 2000 229 128
- JP-A- 2000 511 805
- JP-A- 2004 188 188
- JP-B2- 2 918 262
- US-A- 4 927 408

## Description

### FIELD OF THE INVENTION

The present invention relates to an iontophoresis device for administering positively charged drug ions to a living body by an action of a positive voltage applied to an active electrode structure holding the drug ions, and in particular, to an iontophoresis device with administration efficiency of a drug or drug ions remarkably enhanced.

### BACKGROUND OF THE INVENTION

An iontophoresis device generally includes an active electrode structure holding a drug solution whose active ingredient is dissociated to plus or minus ions (drug ions) and a counter electrode structure that functions as a counter electrode of the active electrode structure. The drug ions are administered to a living body by the application of a voltage with the same polarity as that of the drug ions to the active electrode structure under the condition that both the assemblies are in contact with the skin of the living body (human being or animal).

Herein, the charge supplied to the active electrode structure is consumed by the movement of the drug ions to the living body and the release of biological counter ions (the ions being present in the living body and charged in a conductivity type opposite to that of the drug ions) to the active electrode structure, and the biological counter ions having a small molecular weight (e.g., Na⁺ and C1-) and hence having a large mobility are released mainly. Therefore, the transport number (ratio of the amount of current contributing to the movement of the drug ions among the whole current supplied to the active electrode structure) decreases, which makes it impossible to administer a sufficient amount of drug.

Patent Documents 1 to 10 disclose iontophoresis devices that have solved the above-mentioned problem.

More specifically, in each of the iontophoresis devices in Patent Documents 1 to 10, an active electrode structure is composed of an electrode, a drug holding part placed on a front side (a side facing to the skin) of the electrode, and an ion-exchange membrane that is placed on a front side of the drug holding part and selectively passes ions with the same polarity as that of the drug ions held by the drug holding part, and the drug ions are administered through the ion-exchange membrane, whereby the release of biological counter ions is suppressed to enhance the transport number and the administration efficiency of the drug is enhanced.

In each of the iontophoresis device in Documents 1 to 10, the active electrode structure further includes an electrolyte solution holding part for holding an electrolyte solution in contact with the electrode, and an ion-exchange membrane that is placed on a front side of the electrolyte solution holding part and selectively passes ions having a conductivity type opposite to that of drug ions, and the drug holding part is placed on a front side of the ion-exchange membrane, thereby achieving the additional effects of preventing the drug ions from being decomposed by isolating the drug ions from the electrode and preventing the movement of H⁺ or OH⁻ ions generated at the electrode to the drug holding part and the skin interface of a living body.

Furthermore, Patent Document 11 discloses an invention obtained by further improving the iontophoresis devices disclosed in Patent Documents 1 to 10. Patent Document 11 describes that the administration amount of a drug can be enhanced remarkably by using an ion-exchange membrane in which a porous film composed of a material such as polyolefin, vinyl chloride resin, or fluorine resin is filled with an ion-exchange resin (a resin provided with an ion-exchange function).

Moreover, US 4927408 discloses an electro-transport transdermal system having two current conducting members, respectively a donator and a counter electrode, each one being respectively positioned adjacent to a donator and a counter electrode pad, separated by an insulator. The donor electrode pad comprising an agent reservoir and an electrolyte reservoir separated by a selectively permeable membrane, which can be a microporous polymer, an ion exchange membrane or an hydrogel, depending on the particular needs of the system.

Patent Document 1: JP 3030517 B
Patent Document 2: JP 2000-229128 A
Patent Document 3: JP 2000-229129 A
Patent Document 4: JP 2000-237326 A
Patent Document 5: JP 2000-237327 A
Patent Document 6: JP 2000-237328 A
Patent Document 7: JP 2000-237329 A
Patent Document 8: JP 2000-288097 A
Patent Document 9: JP 2000-288098 A
Patent Document 10: WO 03/037425
Patent Document 11: JP 2004-188188 A

### DISCLOSURE OF INVENTION

### PROBLEMS TO BE SOLVED

As described above, the iontophoresis device disclosed in Patent Document 11 is considered to be the one having the most excellent administration efficiency of a drug among those which are known at present. The present invention provides an iontophoresis device having the features of claim 1 with administration efficiency of a drug further dramatically enhanced, even compared with the iontophoresis device disclosed in Patent Document 11. Advantageous embodiments of the present invention are described in the respective claims.

### MEANS FOR SOLVING PROBLEMS

The present invention provides an iontophoresis device as defined in claim 1.

The present invention provides an iontophoresis device for administering positively charged drug ions through a cellulose-based resin film, including an active electrode structure having an electrode to which a positive voltage is applied, a drug holding part for holding a drug solution containing drug ions, the drug holding part being placed on the front side of the electrode facing the skin or mucous membrane, and a cellulose-based resin film placed on the front side of the drug holding part facing the skin or mucous membrane.

More specifically, the present invention provides an iontophoresis device for administering a drug whose active ingredient is dissociated to plus ions in a solution, in which a cellulose-based resin film is used in place of the ion-exchanged film placed on the front side of the drug holding part facing the skin or mucous membrane in each of the iontophoresis devices in Patent Documents 1 to 11.

The cellulose-based resin film is known to have a function as a cation exchange membrane. However, the characteristics such as an ion-exchange ability of the cellulose-based resin film are inferior to those of generally used cation exchange membranes (e.g., those illustrated in Patent Documents 1 to 11). Accordingly, it has been out of consideration for those skilled in the art to apply the cellulose-based resin film to the iontophoresis device.

In fact, in the study by the inventors of the present invention, the characteristics superior to those of the other cation exchange membranes have not been confirmed in vitro evaluation, which is generally performed in an initial stage of development. However, when evaluation was performed in vivo using a living body, it has been found that, according to the above-mentioned iontophoresis device of the present invention, the administration efficiency of a drug (drug administration amount per unit time under the same current conditions from a film surface with the same surface area) is remarkably enhanced, even compared with the iontophoresis device using a cation exchange resin disclosed in Patent Document 11.

Herein, examples of the drug whose active ingredient is dissociated to plus ions in the present invention include: an anesthetic agent such as morphine hydrochloride or lidocaine; a gastrointestinal disease therapeutic agent such as carnitine chloride; and a skeletal muscle relaxant such as pancuronium bromide.

Furthermore, the drug holding part in the present invention can be configured as a container for holding the above-mentioned drug solution in a liquid state. The drug holding part may hold the drug solution gelled or gelatinized with an appropriate gelling agent. Alternatively, a polymer carrier or the like impregnated with a drug solution may be used as the drug holding part.

The cellulose-based resin film in the present invention is a thin film composed of a cellulose-based resin such as regenerated cellulose, cellulose ester, cellulose ether, or cellulose nitrate. Further, a thin film composed of a cellulose-based resin blended or mixed with other components (resin, plasticizer, cross-linker, etc.) can also be used as the cellulose-based resin film of the present invention, as long as a main component is the cellulose-based resin, and a serious damage to the administration characteristics (administration efficiency, safety, etc.) of a drug, which impairs the use as an iontophoresis device, is not caused.

Furthermore, the cellulose-based resin film of the present invention is preferably a porous film with an appropriate pore size in accordance with the molecular weight of drug ions to be administered. The average pore diameter is typically 10⁻¹⁰m (1 Å) to several µm, more preferably 10⁻¹⁰m to 10⁻⁷m (1 to 1,000 Å), and particularly preferably 10⁻¹⁰m to 10⁻⁸m (1 to 100 Å).

Furthermore, the iontophoresis device of the present invention uses the active electrode structure under the condition that it is attached to the skin of a living body. Therefore, it is desired that the cellulose-based resin film used herein have flexibility capable of following the expansion/contraction and bending of the skin of the living body and a strength to such a degree not to be broken with a stress caused by such expansion/contraction and bending. Generally, when the thickness of the cellulose-based resin film increases, the strength can be enhanced, while the flexibility is lost. Therefore, it is preferable that an appropriate thickness be selected in conjunction with the above-mentioned both characteristics in accordance with the kind of the cellulose-based resin film.

Furthermore, the cellulose-based resin film of the present invention can incorporate a cation exchange group such as a sulfonic acid group, a carboxylic acid group, or a phosphonic acid group by the action of chlorosulfonic acid, chloracetic acid, an inorganic cyclic triphosphate, or the like. This can further enhance the transport number of drug ions in the administration of a drug, and further enhance the administration efficiency of a drug.

Alternatively, a cellulose-based resin film filled with ion-exchange resin with a cation exchange group introduced thereto can also be used as the cellulose-based resin film of the present invention. This also enhances the transport number of drug ions in the administration of a drug, and further increases the administration efficiency of a drug.

Such a cellulose-based resin film can, for example, be obtained by: impregnating a porous thin film composed of cellulose-based resin with a monomer composition composed of a hydrocarbon type monomer having a function group capable of introducing a cation exchange group, a cross-linkable monomer, and a polymerization initiator; and allowing chlorosulfonic acid, chloracetic acid, an inorganic cyclic triphosphate, etc. to act on the resultant porous thin film body.

A sulfonic acid group that is a strong acid group is most preferable as the cation exchange group to be introduced to the above-mentioned cellulose-based resin film or ion-exchange resin.

Furthermore, each of the above cation exchange groups may be present as a free acid, or may be present as a salt with alkaline metal ions such as sodium ions and potassium ions, ammonium ions, etc.

The present invention can also be an iontophoresis device, including an active electrode structure having: an electrode to which a positive voltage is applied; a drug holding part for holding a drug solution containing positively charged drug ions, the drug holding part being placed on the front side of the electrode facing the skin or mucous membrane; and a complex film composed of a cation exchange membrane and a cellulose-based resin film placed on the front side of the cation exchange membrane facing the skin or mucous membrane, the complex film being placed on the front side of the drug holding part facing the skin or mucous membrane, in which the drug ions are administered through the cellulose-based resin film. This can further enhance the transport number in the administration of a drug, and further enhance the administration efficiency of a drug.

The same film as mentioned above can also be used as the cellulose-based resin film in this invention.

In this case, it is preferable to use as the cation exchange membrane a configuration filled with an ion-exchange resin in which a cation exchange group is introduced to a porous film made of a material such as polyolefin, a vinyl chloride resin, or a fluorine resin. This can further enhance the transport number in the administration of a drug.

In the above-mentioned complex film, in order to prevent an air layer from being present at an interface between the cation exchange membrane and the cellulose-based resin film, it is preferable to bond the interface between them so as to integrate the cation exchange membrane and the cellulose-based resin film.

Examples of a bonding method include adhesion by heat sealing, ultrasonic bonding, adhesion with an adhesive such as a cyanoacrylate-type adhesive, and a cross-linking reaction with a cross-linker such as divinylbenzene. Alternatively, a cellulose-based resin film is formed on a cation exchange membrane (e.g., cellulose is regenerated by allowing sulfuric acid to act on a cellulose copper ammonia solution applied to a cation exchange membrane), whereby the cation exchange membrane can be bonded to the cellulose-based resin film.

Herein, in the case of bonding the cation exchange membrane to the cellulose-based resin film by the adhesion, cross-linking reaction, or formation of a cellulose-based resin film on a cation exchange membrane, it is preferable to perform bonding under the condition that at least the surface of a cation exchange membrane facing the cellulose-based resin film is roughened by an approach such as embossing, grooving, notching, mechanical polishing, or chemical polishing. This can enhance the adhesion and integration of the cation exchange membrane and the cellulose-based resin film.

Furthermore, the cation exchange membrane can also be roughened by mixing an inorganic filler such as calcium carbonate or magnesium carbonate, or an organic filler such as denatured polyethylene particles or denatured polyacrylic acid resin particles, with a resin film constituting the cation exchange membrane.

### BREIF DESCRIPTION OF THE DRAWINGS

[Fig. 1] FIG. 1 illustrates a configuration of an iontophoresis device according to one embodiment of the present invention;
[Fig. 2] FIG. 2 illustrates a configuration of an iontophoresis device according to another embodiment of the present invention;
[Fig. 3] FIG. 3 shows a time transition (a) of the concentration of morphine in the blood and a pH value (b) of a drug solution and an electrolyte solution before and after the administration of a drug, when morphine hydrochloride is administered to a mouse using the iontophoresis device according to the present invention;
[Fig. 4] FIG. 4 shows a time transition of the concentration of morphine in the blood, when morphine hydrochloride is administered to a mouse using a conventional iontophoresis device;
[Fig. 5] FIG. 5 illustrates a configuration of a test device used for evaluating morphine transfer characteristics in vitro;
[Fig. 6] FIG. 6 shows evaluation results of morphine transfer characteristics in a test device equivalent to the iontophoresis device of the present invention; and
[Fig. 7] FIG. 7 shows evaluation results of morphine transfer characteristics in a test device equivalent to a conventional iontophoresis device.

### BEST MODE FOR CARRYING OUT THE INVENTION

Hereinafter, an embodiment of the present invention will be described with reference to the drawings.

As shown, an iontophoresis device X1 of the present invention includes an active electrode structure 1, a counter electrode structure 2, and a power source 3, as main components (members). Reference numeral 4 denotes the skin (or a mucous membrane).

The active electrode structure 1 includes an electrode 11 connected to a positive pole of the power source 3, an electrolyte solution holding part 12 for holding an electrolyte solution in contact with the electrode 11, an anion exchange membrane 13 placed on a front side of the electrolyte solution holding part 12 facing the skin or mucous membrane 4, a drug holding part 14 placed on a front side of the anion exchange membrane 13 facing the skin or mucous membrane 4, and a cellulose-based resin film 15 placed on the front side of the drug holding part 14 facing the skin or mucous membrane 4. The entire active electrode structure 1 is housed in a cover or a container 16 composed of a material such as a resin film or a plastic.

On the other hand, the counter electrode structure 2 includes an electrode 21 connected to a negative pole of the power source 3, an electrolyte solution holding part 22 for holding an electrolyte solution in contact with the electrode 21, a cation exchange membrane 23 placed on a front side of the electrolyte solution holding part 22 facing the skin or mucous membrane 4, an electrolyte solution holding part 24 placed on a front side of the cation exchange membrane 23 facing the skin or mucous membrane 4, and an anion exchange membrane 25 placed on a front side of the electrolyte solution holding part 24 facing the skin or mucous membrane 4. The entire counter electrode structure 2 is housed in a cover or a container 26 composed of a material such as a resin film or a plastic.

In the iontophoresis device X1, those which are made of any conductive material can be used as the electrodes 11 and 21 without any particular limit. In particular, an inactive electrode composed of carbon, platinum, or the like is used preferably, and a carbon electrode free from the elution of metal ions and the transfer thereof to a living body can be used more preferably.

However, an active electrode such as a silver/silver chloride couple electrode in which the electrode 11 is made of silver and the electrode 21 is made of silver chloride can also be adopted.

For example, in the case of using the silver/silver chloride couple electrode, in the electrode 11 that is a positive pole, a silver electrode and chlorine ions (Cl⁻) easily react with each other to generate insoluble AgCl as represented by Ag⁺Cl⁻→AgCl+e⁻, and in the electrode 21 that is a negative pole, chlorine ions (C1-) are eluted from a silver chloride electrode. Consequently, the following effects can be obtained: the electrolysis of water is suppressed, and the rapid acidification based on H⁺ ions at the positive pole, and the rapid basification based on OH- ions at the negative pole can be prevented.

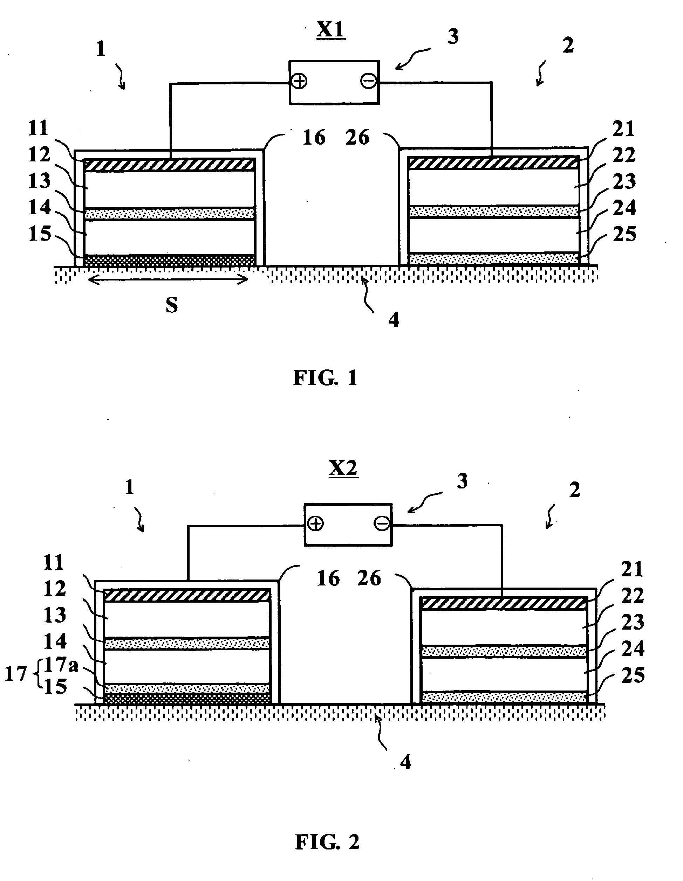

In contrast, in the active electrode structure 1 and the counter electrode structure 2 in the iontophoresis device X1 in FIG. 1, owing to the function of the anion exchange membrane 13 and the cation exchange membrane 23, the rapid acidification based on H⁺ ions in the electrolyte solution holding part 12 and the rapid basification based on OH⁻ ions in the electrolyte solution holding part 22 are suppressed. Therefore, an inexpensive carbon electrode free from the elution of metal ions can be used preferably in place of the active electrode such as a silver/silver chloride couple electrode.

Furthermore, the electrolyte solution holding parts 12, 22, and 24 in the iontophoresis device X1 in FIG. 1 hold an electrolyte solution so as to keep the conductivity. Phosphate buffered saline, physiological saline, etc. can be used as the electrolyte solution typically.

Furthermore, in order to more effectively prevent the generation of gas caused by the electrolytic reaction of water and the increase in a conductive resistance caused by the generation of gas, or the change in pH caused by the electrolytic reaction of water, an electrolyte that is more readily to be oxidized or reduced than the electrolytic reaction (oxidation at the positive pole and the reduction at the negative pole) of water can be added to the electrolyte solution holding parts 12 and 22. In terms of the biological safety and economic efficiency (low cost and easy availability), for example, an inorganic compound such as ferrous sulfate or ferric sulfate, a medical agent such as ascorbic acid (vitamin C) or sodium ascorbate, and an organic acid such as lactic acid, oxalic acid, malic acid, succinic acid, or fumaric acid and/or a salt thereof can be used preferably. Alternatively, a combination of those substances (for example, 1:1 mixed aqueous solution containing 1 mol (M) of lactic acid and 1 mol (M) of sodium fumarate) can also be used.

The electrolyte solution holding parts 12, 22, and 24 may hold the above-mentioned electrolyte solution in a liquid state. However, the electrolyte solution holding parts 12, 22, and 24 may be configured by impregnating a water-absorbing thin film carrier made of a polymer material or the like with the above-mentioned electrolyte solution, thereby enhancing the ease of handling thereof. The same thin film carrier as that can be used in the drug holding part 14 can be used as the thin film carrier used herein. Therefore, the detail thereof will be described in the following description regarding the drug holding part 14.

The drug holding part 14 in the iontophoresis device X1 according to this embodiment holds at least an aqueous solution of a drug whose active ingredient is dissociated to plus drug ions by the dissolution, as a drug solution.

Herein, the drug holding part 14 may hold a drug solution in a liquid state. However, it is also possible to impregnate such a water-absorbing thin film carrier as described below with a drug solution so as to enhance the ease of handling thereof.

Examples of a material that can be used for the water-absorbing thin film carrier in this case include a hydrogel body of acrylic resin (acrylhydrogel film), segmented polyurethane gel film, and an ion conductive porous sheet for forming a gel solid electrolyte. By impregnating the above aqueous solution at an impregnation ratio of 20 to 60%, a high transport number (high drug delivery property), e.g., 70 to 80% can be obtained.

The impregnation ratio in the present specification is represented by % by weight (i.e., 100*(W-D)/D [%] where D is a weight in a dry state and W is a weight after impregnation). The impregnation ratio should be measured immediately after the impregnation with an aqueous solution to eliminate a chronological influence.

Furthermore, the transport number refers to the ratio of the amount of current contributing to the transfer of particular ions among the whole current flowing through the electrolyte solution. In the present specification, the transport number is used in terms of that regarding drug ions, i.e., the ratio of a current contributing to the transfer of drug ions among the whole currents supplied to the active electrode structure.

Herein, the above-mentioned acrylhydrogel film (for example, available from Sun Contact Lens Co., Ltd.) is a gel body having a three-dimensional network structure (cross-linking structure). When an electrolyte solution that is a dispersion medium is added to the acrylhydrogel film, the acrylhydrogel film becomes a polymer adsorbent having ion conductivity. Furthermore, the relationship between the impregnation ratio of the acrylhydrogel film and the transport number can be adjusted by controlling the size of the three-dimensional network structure and the kind and ratio of a monomer constituting a resin. The acrylhydrogel film with an impregnation ratio of 30 to 40% and a transport number of 70 to 80% can be prepared from 2-hydroxyethylmethacrylate and ethyleneglycol dimethacrylate (monomer ratio 98 to 99.5 : 0.5 to 2), and it is confirmed that the impregnation ratio and transport number are almost the same in a range of an ordinary thickness of 0.1 to 1 mm.

Furthermore, the segmented polyurethane gel film has, as segments, polyethylene glycol (PEG) and polypropylene glycol (PPG), and can be synthesized from a monomer and diisocyanate constituting these segments. The segmented polyurethane gel film has a three-dimensional structure cross-linked by a urethane bond, and the impregnation ratio, transport number, and adhesion strength of the gel film can be easily adjusted by controlling the size of a network, and the kind and ratio of a monomer in the same way as in the acrylhydrogel film. When water that is a dispersion medium and an electrolyte (alkaline metal salt, etc.) are added to the segmented polyurethane gel film (porous gel film), oxygen in an ether connecting part of polyether forming a segment and an alkaline metal salt form a complex, and ions of the metal salt move to oxygen in a subsequent blank ether connecting part when a current flows, whereby the conductivity is expressed.

As the ion conductive porous sheet for forming a gel solid electrolyte, for example, there is the one disclosed in JP 11-273452 A. This porous sheet is based on an acrylonitrile copolymer, and a porous polymer with a porosity of 20 to 80%. More specifically, this porous sheet is based on an acrylonitrile copolymer with a porosity of 20 to 80% containing 50 mol% or more (preferably 70 to 98 mol%) of acrylonitrile. The acrylonitrile gel solid electrolytic sheet (solid-state battery) is prepared by impregnating an acrylonitrile copolymer sheet soluble in a non-aqueous solvent and having a porosity of 20 to 80%, with a non-aqueous solvent containing an electrolyte, followed by gelling, and a gel body includes a gel to a hard film.

In terms of the ion conductivity, safety, and the like, the acrylonitrile copolymer sheet soluble in a non-aqueous solvent is preferably composed of an acrylonitrile/C1 to C4 alkyl (meth)acrylate copolymer, an acrylonitrile/vinylacetate copolymer, an acrylonitrile/styrene copolymer, an acrylonitrile/vinylidene chloride copolymer, or the like. The copolymer sheet is made porous by an ordinary method such as a wet (dry) paper making method, a needlepunching method that is a kind of a non-woven fabric producing method, a water-jet method, drawing perforation of a melt-extruded sheet, or perforation by solvent extraction. In the present invention, among the above-mentioned ion conductive porous sheets of an acrylonitrile copolymer used in a solid-state battery, a gel body (a gel to a hard film) holding the above-mentioned aqueous solution in a three-dimensional network of a polymer chain and in which the above-mentioned impregnation ratio and transport number are achieved is useful as a thin film carrier used in the drug holding part 14 or the electrolyte solution holding parts 12, 22, and 24 of the present invention.

In the present invention, regarding the conditions for impregnating the above-mentioned thin film carrier with a drug solution or an electrolyte solution, the optimum conditions may be determined in terms of the impregnation amount, impregnation speed, and the like. For example, an impregnation condition of 30 minutes at 40°C may be selected.

An ion-exchange membrane carrying an ion-exchange resin having an anion exchange function in a base, for example, NEOSEPTA, AM-1, AM-3, AMX, AHA, ACH, ACS, ALE04-2, AIP-21, produced by Tokuyama Co., Ltd. can be used as the anion exchange membrane (ion-exchange membrane having characteristics of selectively passing minus ions) 13 and 25 in the iontophoresis device X1 according to this embodiment. An ion-exchange membrane carrying an ion-exchange resin having a cation exchange function in a base, for example, NEOSEPTA, CM-1, CM-2, CMX, CMS, CMB, CLE04-2, produced by Tokuyama Co., Ltd. can be used as the cation exchange membrane (ion-exchange membrane having characteristics of selectively passing plus ions) 23. In particular, a cation exchange membrane in which a part or an entirety of a pore of a porous film is filled with an ion-exchange resin having a cation exchange function, or an anion exchange membrane filled with an ion-exchange resin having an anion exchange function can be used preferably.

Herein, a fluorine type resin with an ion-exchange group introduced to a perfluorocarbon skeleton or a hydrocarbon type resin containing a resin that is not fluorinated as a skeleton can be used as the above-mentioned ion-exchange resin. In view of the convenience of a production process, a hydrocarbon type ion-exchange resin is preferable. Furthermore, although the filling ratio of the ion-exchange resin is also related to the porosity of the porous film, the filling ratio is generally 5 to 95% by mass, in particular, 10 to 90% by mass, and preferably 20 to 60% by mass.

Furthermore, there is no particular limit to an ion-exchange group of the above-mentioned ion-exchange resin, as long as it is a functional group generating a group having negative or positive charge in an aqueous solution. As specific examples of the functional group to be such an ion-exchange group, those of a cation exchange group include a sulfonic acid group, a carboxylic acid group, and a phosphonic acid group. Those acid groups may be present in the form of a free acid or a salt. Examples of a counter cation in the case of a salt include alkaline metal cations such as sodium ions and potassium ions, and ammonium ions. Of those cation exchange groups, generally, a sulfonic acid group that is a strong acidic group is particularly preferable. Furthermore, examples of the anion exchange group include primary to tertiary amino groups, a quaternary ammonium group, a pyridyl group, an imidazole group, a quaternary pyridinium group, and a quaternary imidazolium group. Examples of a counter anion in those anion exchange groups include halogen ions such as chlorine ions and hydroxy ions. Of those anion exchange groups, generally, a quaternary ammonium group and a quaternary pyridinium group that are strong basic groups are used preferably.

Furthermore, a film shape or a sheet shape having a number of small holes passing from front to back sides are used as the above-mentioned porous film without any particular limit. In order to satisfy both the high strength and the flexibility, it is preferable that the porous film be made of a thermoplastic resin.

Examples of the thermoplastic resins constituting the porous film include, without limitation: polyolefin resins such as homopolymers or copolymers of α-olefins such as ethylene, propylene, 1-butene, 1-pentene, 1-hexene, 3-methyl-1-butene, 4-methyl-1-pentene, and 5-methyl-1-heptene; vinyl chloride resins such as polyvinyl chloride, vinyl chloride-vinyl acetate copolymers, vinyl chloride-vinylidene chloride copolymers, and vinyl chloride-olefin copolymers; fluorine resins such as polytetrafluoroethylene, polychlorotrifluoroethylene, polyvinylidene fluoride, tetrafluoroethylene-hexafluoropropylene copolymers, tetrafluoroethylene-perfluoroalkyl vinylether copolymers, and tetrafluoroethylene-ethylene copolymers; polyamide resins such as nylon 6 and nylon 66; and those which are made from polyamide resins. Polyolefin resins are preferably used as they are superior in mechanical strength, flexibility, chemical stability, and chemical resistance, and have good compatibility with ion-exchange resins. As the polyolefin resins, polyethylene and polypropylene are particularly preferable and polyethylene is most preferable.

There is no particular limit to the property of the above-mentioned porous film made of the thermoplastic resin. However, the average pore diameter of pores may be preferably 0.005 to 5.0 µ m, more preferably 0.01 to 2.0 µ m, and most preferably 0.02 to 0.2 µ m since the porous film having such an average pore diameter is likely to be a thin ion-exchange membrane having excellent strength and a low electric resistance. The average pore diameter in the present specification refers to an average flow pore diameter measured in accordance with a bubble point method (JIS K3832-1990). Similarly, the porosity of the porous film may be preferably 20 to 95%, more preferably 30 to 90%, and most preferably 30 to 60%. Furthermore, the thickness of the porous film may be preferably 5 to 140 µm, more preferably 10 to 120 µm, and most preferably 15 to 55 µm. Usually, an anion exchange membrane or a cation exchange membrane using such a porous film has a thickness of the porous film with +0 to 20 µm.

The cellulose-based resin film 15 used in the iontophoresis device X1 according to this embodiment can be constituted by cellulose-based resins such as cellulose esters (e.g., cellulose acetate, cellulose propionate, cellulose acetate butyrate or regenerated cellulose manufactured by a method such as a cuprammonium process or a tertiary amineoxide process), cellulose ethers (e.g., hydroxyethyl cellulose or hydroxypropyl cellulose) or nitrocellulose. A porous thin film of cellulose-based resins having an average pore diameter of preferably 10⁻¹⁰m (1 Å) to a few µm, more preferably 10⁻¹⁰m to 10⁻⁷m (1 to 1,000 Å), and particularly preferably around 10⁻¹⁰m to 10⁻⁸m (1 to 100 Å), and a thickness of preferably 10 to 200 µm, and particularly preferably 20 to 50 µm can be used.

A cation exchange group such as a sulfonic acid group, a carboxylic acid group, or a phosphonic acid group can be introduced to the above-mentioned cellulose-based resin film by allowing chlorosulfonic acid, chloroacetic acid, inorganic cyclic triphosphate, or the like to act on the cellulose-based resin film. By using a cellulose-based resin film with such a cation exchange group introduced thereto as the cellulose-based resin film 15, the administration efficiency of a drug can be enhanced further.

Alternatively, a porous thin film made of the above-mentioned cellulose-based resin in which pores are filled with a cation exchange resin can also be used for the cellulose-based resin film 15.

The cellulose-based resin film filled with a cation exchange resin can be obtained by: impregnating the above-mentioned porous thin film made of a cellulose-based resin with a monomer composition composed of a hydrocarbon type monomer having a functional group capable of introducing a cation exchange group, a cross-linkable monomer, and a polymerization initiator; polymerizing them under appropriate reaction conditions; and allowing chlorosulfonic acid, chloracetic acid, an inorganic cyclic triphosphate, or the like to act on the resultant porous thin film.

Examples of the hydrocarbon-type monomer having a functional group capable of introducing a cation exchange group include aromatic vinyl compounds such as styrene, α-methylstyrene, 3-methylstyrene, 4-methylstyrene, 2,4-dimethylstyrene, p-tert-butylstyrene, α-halogenated styrene, and vinylnaphthalene and each one or more of them can be used. Examples of an available cross-linkable monomer include: polyfunctional vinyl compounds such as divinylbenzenes, divinyl sulfone, butadiene, chloroprene, divinylbiphenyl, and trivinylbenzene; and polyfunctional methacrylic acid derivatives such as trimethylol methane trimethacrylate, methylenebis acrylamide, and hexamethylene methacrylamide. Examples of an available polymerization initiator include octanoyl peroxide, lauroyl peroxide, t-butylperoxy-2-ethylhexanoate, benzoyl peroxide, t-butyl peroxyisobutyrate, t-butyl peroxylaurate, t-hexyl peroxybenzoate, and di-t-butylperoxide.

In addition to the above components, other hydrocarbon-type monomers which are copolymerizable with the above hydrocarbon-type monomers and cross-linkable monomers, or plasticizers may be added as required. Examples of the other monomers which may be used include acrylonitrile, acrolein, and methylvinylketone. Further, examples of the plasticizers which may be used include dibutyl phthalate, dioctyl phthalate, dimethyl isophthalate, dibutyladipate, triethylcitrate, acetyltributylcitrate, dibutylsebacate, and dibenzylether.

A battery, a voltage stabilizer, a current stabilizer (galvano device), a voltage/current stabilizer, or the like can be used as the power source 3 in the iontophoresis device of the present invention. It is preferable to use a current stabilizer that is operated under safe voltage conditions in which an arbitrary current can be adjusted in a range of 0.01 to 1.0 mA, preferably 0.01 to 0.5 mA, specifically, at 50V or less, preferably, 30 V or less.

The iontophoresis device X1 1 according to this embodiment has remarkably higher administration efficiency of a drug than that of a conventional iontophoresis device using a cation exchange membrane in place of the cellulose-based resin film 15, as described later in examples.

FIG. 2 illustrates a configuration of an iontophoresis device X2 according to another embodiment of the present invention.

As shown in FIG. 2, the iontophoresis device X2 has the same configuration as that of the above-mentioned iontophoresis device X1, except that a complex film 17 made of a cation exchange membrane 17a placed on the front side of the drug holding part 14 facing the skin or mucous membrane 4 and a cellulose-based resin film 15 placed on a front side of the cation exchange membrane 17a facing the skin or mucous membrane 4 is provided, in place of the cellulose-based resin film 15.

The same cation exchange membrane as that described with respect to the cation exchange membrane 23 can be used as the cation exchange membrane 17a of the complex film 17. The same cellulose-based resin film as that described with respect to the cellulose-based resin film 15 can be used as the cellulose-based resin film 15.

In order to prevent an air layer from being present at an interface between the cation exchange membrane 17a and the cellulose-based resin film 15, it is preferable that the complex film 17 be formed by bonding the interface between the cation exchange membrane 17a and the cellulose-based resin film 15 by heat sealing, ultrasonic bonding, adhesion with an adhesive, chemical bonding with a cross-linker, or formation of the cellulose-based resin film 15 on the cation exchange membrane 17a. In the case of bonding by means of the adhesion, chemical bonding, or the like, in order to make the integration and adhesion of the bonding satisfactory, it is preferable to use the cellulose-based resin film 15 in which at least the connection side surface is roughened by an approach such as embossing, grooving, notching, mechanical polishing, or chemical polishing, or by mixing an inorganic filler (such as calcium carbonate or magnesium carbonate) or an organic filler (such as denatured polyethylene particles or denatured polyacrylic acid resin particles) with a cellulose-based resin.

The condition of heat sealing and ultrasonic bonding, the kind and adhesion condition of an adhesive, the kind and cross-linking condition of a cross-linker, and the like can be appropriately determined depending upon the kind of the cation exchange membrane 17a (mainly, the kind of a porous resin film used in the cation exchange membrane 17a) and the kind of the cellulose-based resin film 15. The bonding herein has an object of preventing the administration efficiency of a drug from decreasing due to the presence of an air layer at the interface between the cation exchange membrane 17a and the cellulose-based resin film 15. Therefore, the bonding only needs to be performed with strength to such a degree that the interface will not be peeled off due to the expansion/contraction and bending of the skin while the iontophoresis device is mounted.

In the iontophoresis device X2 according to this embodiment, the ion-exchange ability of the complex film 17 is enhanced by the cation exchange membrane 17a, so that the transport number in the administration of a drug can be increased, and the administration efficiency of a drug comparable to or higher than that of the iontophoresis device X1 can be obtained.

### Example 1 (in vivo test 1)

Using a C57BL/6 mouse (male) of 20 to 24 weekly age as a test animal, an administration test of morphine hydrochloride in the above-mentioned iontophoresis device X1 was performed.

NEOSEPTA ALE04-2 produced by Tokuyama Co., Ltd. was used as each of the anion exchange membranes 13 and 25 of the iontophoresis device X1. NEOSEPTA CLE04-02 produced by Tokuyama Co., Ltd. was used as the cation exchange membrane 23. A regenerated cellulose dialysis membrane UC8-32-25 (average pore diameter: 5*10⁻⁹m (50 A), transmission molecular weight (MWCO): about 14,000, film thickness: 50 µm) of 99% a -cellulose obtained from Viskase Sales Co. (Illinois in the US) was used as the cellulose-based resin film 15. 50 mg/mL of morphine hydrochloride was used as a drug solution of the drug holding part 14. A 7 : 1 mixed solution of 0.7 mol/L sodium fumarate aqueous solution and 0.7 mol/L lactic acid aqueous solution was used as an electrolyte solution of the electrolyte solution holding parts 12, 22, and 24. The effective area of the active electrode structure 1 (area of a film surface of the cellulose-based resin film 15 through which a drug is administered/see the reference S in FIG. 1) was 2.23 cm2.

The drug was administered under the condition that the active electrode structure 1 and the counter electrode structure 2 were brought into contact with different sites of the shaved abdomen of the mouse, and a constant current was allowed to flow continuously at 0.45 mA/cm2 for 120 minutes.

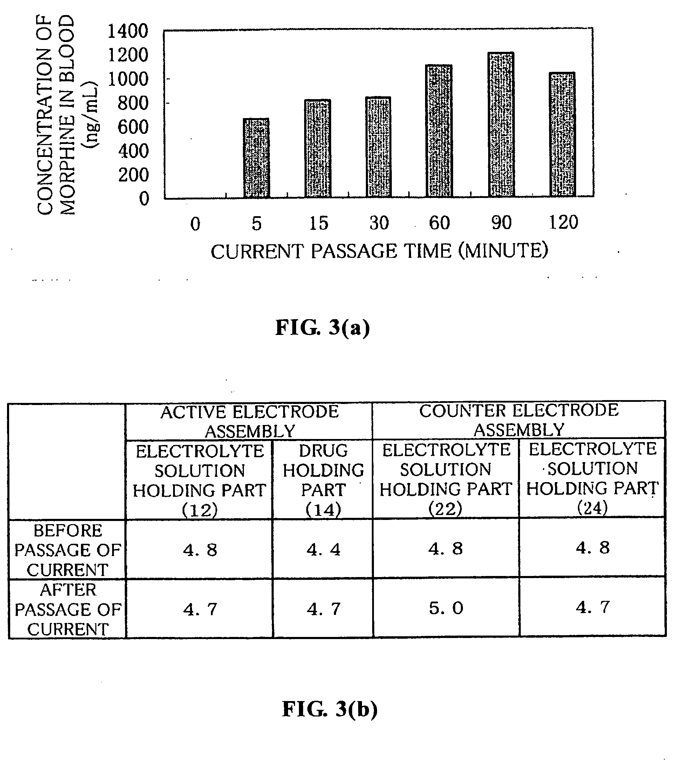

FIG. 3(a) shows the transition of the concentration of morphine in the blood of the mouse during the passage of a current under the above-mentioned conditions, and FIG. 3(b) shows the pH values of the electrolyte solution of the electrolyte solution holding parts 12, 22, and 24, and the drug solution of the drug holding part 14 before the commencement of the passage of a current and after the completion thereof.

### Comparative Example 1 (in vivo test 2)

Using an iontophoresis device with the same configuration as that of the iontophoresis device X1 of Example 1 except for using a cation exchange membrane (NEOSEPTA CLE04-2 produced by Tokuyama Co., Ltd.) in place of the cellulose-based resin film 15, morphine hydrochloride was administered to the mouse under the same conditions as those of Example 1.

Here, NEOSEPTA ALE04-2 that is an anion exchange membrane and CLE04-2 that is a cation exchange membrane are ion-exchange membranes each having a configuration in which a pore of a porous film is filled with an ion-exchange resin. Therefore the iontophoresis device used in Comparative Example 1 has the same configuration as that of the iontophoresis device of Patent Document 11 that is considered to exhibit the highest administration efficiency of a drug in the prior art.

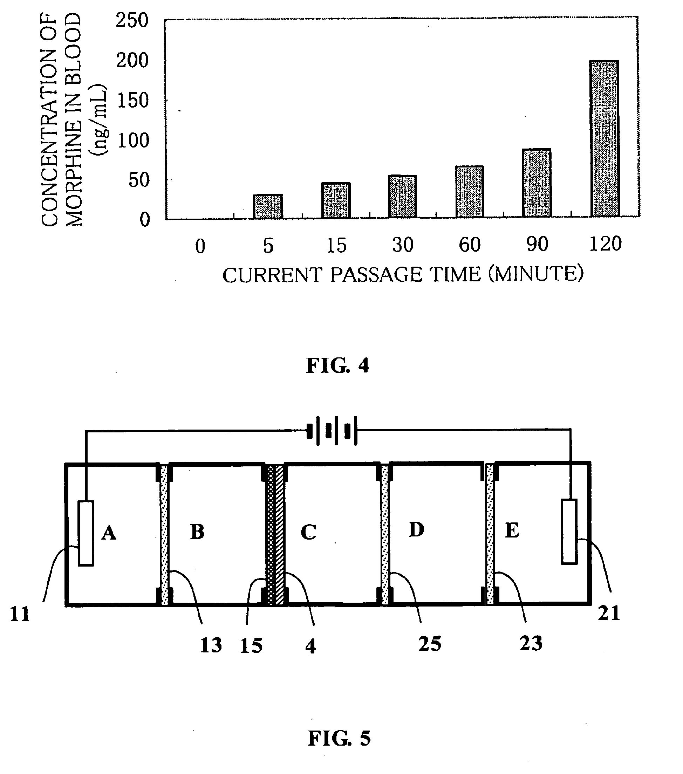

FIG. 4 shows the transition of the concentration of morphine in the blood of the mouse during the passage of a current in Comparative Example 1.

### Reference Example 1 (in vitro test 1)

A test device having a configuration equivalent to that of the iontophoresis device X1 used in Example 1 was produced, and a constant current was allowed to flow continuously at 0.45 mA/ cm² for 120 minutes.

FIG. 5 illustrates the configuration of the test device. In FIG. 5, Reference numerals 11 and 21 denote electrodes. Reference numerals 13 and 25 denote anion exchange membranes (NEOSEPTA ALE04-2 produced by Tokuyama Co., Ltd.). Reference numeral 23 denotes a cation exchange membrane (NEOSEPTA CLE04-2 produced by Tokuyama Co., Ltd.). Reference numeral 15 denotes a cellulose-based resin film (dialysis membrane UC8-32-25 produced by Viskase Sales Co.). Reference numeral 4 denotes the skin collected from a mouse. An A-chamber, a D-chamber and an E-chamber are filled with a mixed solution (7 : 1) of 0.7 mol/L sodium fumarate aqueous solution and 0.7 mol/L lactic acid aqueous solution as an electrolyte solution. A B-chamber is filled with 50 mg/mL of morphine hydrochloride as a drug solution. A C-chamber is filled with physiological saline.

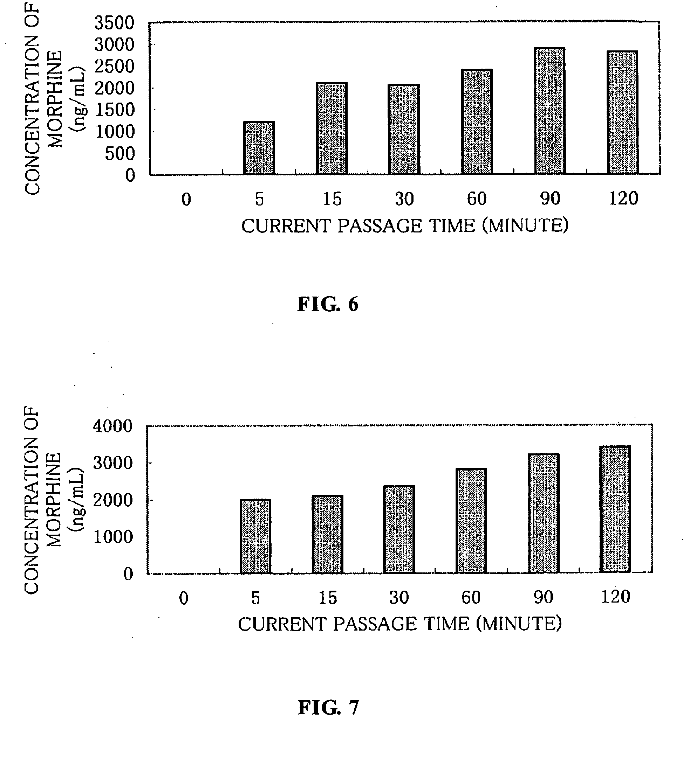

FIG. 6 shows the transition of the concentration of morphine in the C-chamber during the passage of a current in Reference Example 1.

### Comparative Reference Example 1 (in vitro test 2)

Using the same test device as that of Reference Example 1 except for using a cation exchange membrane (NEOSEPTA CLE04-2 produced by Tokuyama Co., Ltd.) in place of the cellulose-based resin film 15 in FIG. 5, which has an equivalent configuration to the iontophoresis device used in Comparative Example 1, a constant current was allowed to flow continuously at 0.45 mA/ cm² for 120 minutes.

FIG. 7 shows the transition of the concentration of morphine in the C-chamber during the passage of a current in Comparative Reference Example 1.

As is apparent from the comparison between FIG. 3(a) and FIG. 4, in the iontophoresis device according to the present invention, morphine was administered at efficiency of 5 to 10 times or more, even compared with the iontophoresis device having the configuration of Comparative Example 1 in which the administration efficiency of a drug has been conventionally considered to be the highest.

Furthermore, as shown in FIG. 3(b), in the electrolyte solution in the electrolyte solution holding parts 12, 22, and 24 and the drug solution of the drug holding part 14 of the iontophoresis device according to the present invention, the pH values hardly changed before and after the passage of a current. Thus, it is understood that the safety and stability of the administration of a drug are ensured.

Furthermore, as shown in FIGS. 6 and 7, in the in vitro test, the transfer speed of morphine in the test device (Reference Example 1) with the configuration of the present invention was inferior by about tens of percentages to that of the test device (Comparative Reference Example 1) with the conventional configuration.

In the technical field of the present invention, the evaluation and study in vitro are generally performed without using a living body in a stage of selecting the material of a member of a device and the like. As described above, the effect of using a cellulose-based resin film can only be confirmed by the evaluation in vivo, and can not be confirmed by the evaluation in vitro, and this fact is considered to be a proof of the difficulty in constituting the present invention.

The present invention has been described with reference to the embodiment. The present invention is not limited thereto, and various alterations can be made within the scope of the claims.

For example, in the above embodiment, the case has been described where the active electrode structure includes the electrolyte solution holding part 12 and the anion exchange membrane 13, in addition to the electrode 11, the drug holding part 14, and the cellulose-based resin film 15 (or the complex film 17). However, the electrolyte solution holding part 12 and the ion-exchange membrane 13 can also be omitted. In this case, although the function of suppressing the decomposition of a drug in the vicinity of the electrode 11, the movement of H⁺ ions to the skin interface, the function of suppressing the variation in pH at the skin interface caused by the movement of H⁺ ions, and the like cannot be achieved to such a degree as that in the above-mentioned embodiment, the administration efficiency of a drug to a living body, which is the basic functional effect of the present invention, is achieved similarly, and such an iontophoresis device is also included in the scope of the present invention.

Similarly, regarding the counter electrode structure , the cation exchange membrane 23 and the electrolyte solution holding part 24, or the anion exchange membrane 25 in addition to the cation exchange membrane 23 and the electrolyte solution holding part 24 can be omitted. In this case, although the performance of suppressing the change in pH in a contact surface of the counter electrode structure 2 with respect to the skin 4 cannot be achieved to such a degree as that in the above-mentioned embodiment, the administration efficiency of a drug to a living body, which is the basic functional effect of the present invention, is achieved similarly, and such an iontophoresis device is also included in the scope of the present invention.

Alternatively, it is also possible that the counter electrode structure 2 is not provided in the iontophoresis device, and for example, under the condition that the active electrode structure is brought into contact with the skin of a living body and a part of the living body is brought into contact with a member to be the earth, a drug is administered by applying a voltage to the active electrode structure. Such an iontophoresis device can also similarly enhance the administration efficiency of a drug to a living body, which is the basic functional effect of the present invention, and is included in the scope of the present invention.

Furthermore, in the above embodiment, the case has been described where the active electrode structure, the counter electrode structure, and the power source are configured separately. It is also possible that those elements are incorporated in a single casing or an entire device incorporating them is formed in a sheet shape or a patch shape, whereby the handling thereof is enhanced, and such an iontophoresis device is also included in the scope of the present invention.

## Claims

1. An iontophoresis device (X1; X2), comprising an active electrode structure (1) including:
an electrode (11) to which a positive voltage is applied;
a drug holding part (14) for holding a drug solution containing positively charged drug ions, the drug holding part (14) being placed on the front side of the electrode facing the skin or mucous membrane (4)
**characterized in that** it includes
a cellulose-based resin film (15) placed on the front side of the drug holding part (14) facing the skin or mucous membrane (4),
wherein the drug ions are administered through the cellulose-based resin film (15).

2. An iontophoresis device (X1; X2), according to claim 1, where the cellulose based resin film (15) is placed on a front side of a cation exchange membrane (17a) facing the skin or mucous membrane (4), both forming a complex film (17) and
the complex film (17) placed on the front side of the drug holding part (14) facing the skin or mucous membrane (4),
wherein the drug ions are administered through the cellulose-based resin film (15).

3. An iontophoresis device (X1; X2) according to claim 2, wherein an interface of the cation exchange membrane (17a) and the cellulose-based resin film (15) are bonded, whereby the cation exchange membrane (17a) is integrated with the cellulose-based resin film (15).

4. An iontophoresis device (X1; X2) according to claim 3, wherein:
a surface of the cation exchange membrane (17a) facing the cellulose-based resin film (15) is roughened; and
the interface are bonded by any one of adhesion with an adhesive, a cross-linking reaction with a cross-linker, and formation of the cellulose-based resin film (15) on the cation exchange membrane (17a).

5. An iontophoresis device (X1; X2) according to any one of claims 2 to 4, wherein the cation exchange membrane (17a) has a structure in which a pore of a porous film is filed with an ion-exchange resin.

6. An iontophoresis device (X1; X2) according to any one of claims 1 to 5, wherein a cation exchange group is introduced to the cellulose-based resin film (15).

7. An iontophoresis device (X1; X2) according to any one of claims 1 to 6, wherein the cellulose-based resin film (15) is filled with an ion-exchange resin with a cation exchange group introduced thereto.

8. An iontophoresis device (X1; X2) according to any one of claims 1 to 7, wherein:
the active electrode structure (1) further includes an electrolyte solution holding part (12) for holding an electrolyte solution in contact with the electrode (11), and an anion exchange membrane (13) placed on the front side of the electrolyte solution holding part (12) facing the skin or mucous membrane (4); and
the drug holding part (14) is placed on the front side of the anion exchange membrane (13) facing the skin or mucous membrane (4).

9. An iontophoresis device (X1; X2) according to any one of claims 1 to 8, further comprising a counter electrode structure (2) including:
a second electrode (21) to which a negative voltage is applied;
a second electrolyte solution holding part (22) for holding an electrolyte solution in contact with the second electrode (21);
a second cation exchange membrane (23) placed on the front side of the second electrolyte solution holding part (22) facing the skin or mucous membrane (4);
a third electrolyte solution holding part (24) for holding an electrolyte solution, the third electrolyte solution holding part (24) being placed on the front side of the second cation exchange membrane (23) facing the skin or mucous membrane (4); and
a second anion exchange membrane (25) placed on the front side of the third electrolyte solution holding part (24) facing the skin or mucous membrane (4).

## Patentansprüche

1. Ein Iontophorese-Gerät (X1; X2), umfassend eine Aktivelektrodenstruktur (1), beinhaltend:
- einer Elektrode (11), an welche eine positive Spannung angelegt wird;
- einen Wirkstoffbehälter (14) zum Aufbewahren eine Wirkstofflösung, die positive geladenen Wirkstoffionen enthält, wobei der Wirkstoffbehälter (14) an der Vorderseite der Elektrode angeordnet ist, die der Haut oder der Schleimhautmembran (4) zugewandt ist,
**dadurch gekennzeichnet, dass** sie
- einen Zellulose-basierten Harzfilm (15), der an der Vorderseite des Wirkstoffbehälters (14), der der Haut oder der Schleimhautmembran (4) zugewandt ist, beinhaltet,
- wobei die Wirkstoffionen durch den Zellulose-basierten Harzfilm (15) verabreicht werden.

2. Ein Iontophorese-Gerät (X1; X2) gemäß Anspruch 1, wobei der Zellulose-basierte Harzfilm (15) an der Vorderseite einer Kationen-Austausch-Membran (17a) angeordnet ist, welche der Haut oder der Schleimhautmembran (4) zugewandt ist, wobei beide einen komplexen Film (17) bilden, und
- der komplexe Film (17) an der Vorderseite des Wirkstoffbehälters (14) angeordnet ist, der der Haut oder der Schleimhautmembran (4) zugewandt ist, angeordnet ist,
- wobei die Wirkstoffionen durch den Zellulose-basierten Harzfilm (15) verabreicht werden.

3. Ein Iontophorese-Gerät (X1; X2) gemäß Anspruch 2, wobei eine Grenzfläche der Kationen-Austausch-Membran (17a) und dem Zellulose-basierten Harzfilm (15) miteinander verbunden sind, wobei die Kationen-Austausch-Membran (17a) in den Zellulose-basierten Harzfilm (15) integriert ist.

4. Ein Iontophorese-Gerät (X1; X2) gemäß Anspruch 3, wobei:
- eine Oberfläche der Kationen-Austausch-Membran (17a), die dem Zellulose-basierten Harzfilm (15) zugewandt ist, ist angeraut; und
- die Grenzflächen sind verbunden mittels Kleben mit einem Klebstoff, einer Vernetzung mit einem Vernetzer, oder Bildung des Zellulose-basierten Harzfilms (15) auf der Kationen-Austausch-Membran (17a).

5. Ein Iontophorese-Gerät (X1; X2) gemäß einem der Ansprüche 2 bis 4, wobei die Kationen-Austausch-Membran (17a) eine Struktur aufweist, in welcher eine Pore eines porösen Films mit einem Ionen-Austausch-Harz gefüllt ist.

6. Ein Iontophorese-Gerät (X1; X2) gemäß einem der Ansprüche 1 bis 5, wobei eine Kationen-Austausch-Gruppe in den Zellulose-basierten Harzfilm (15) eingefügt ist.

7. Ein Iontophorese-Gerät (X1; X2) gemäß einem der Ansprüche 1 bis 6, wobei der Zellulose-basierte Harzfilm (15) mit einem Ionen-Austausch-Harz gefüllt ist, in welches eine Kationen-Austausch-Gruppe eingefügt ist.

8. Ein Iontophorese-Gerät (X1; X2) gemäß einem der Ansprüche 1 bis 7, wobei:
- die aktive Elektrodenstruktur (1) beinhaltet ferner ein Elektrolytlösungsaufbewahrungsteil (12) zum Aufbewahren einer Elektrolytlösung in Kontakt mit der Elektrode (11), und eine Anionen-Austausch-Membran (13), welche an der Vorderseite des Elektrolytlösungsaufbewahrungsteils (12) angebracht ist, die der Haut oder der Schleimhautmembran (4) zugewandt ist; und
- der Wirkstoffbehälter (14) ist an der Vorderseite der Anionen-Austausch-Membran (13) angeordnet, die der Haut oder der Schleimhautmembran (4) zugewandt ist.

9. Ein Iontophorese-Gerät (X1; X2) gemäß einem der Ansprüche 1 bis 8, ferner umfassend eine Gegenelektrodenstruktur (2), beinhaltend:
- eine zweite Elektrode (21), an welche eine negative Spannung angelegt wird;
- ein zweites Elektrolytlösungsaufbewahrungsteil (22) zum Aufbewahren einer Elektrolytlösung in Kontakt mit der zweiten Elektrode (21),
- eine zweite Kationen-Austausch-Membran (23), angeordnet an der Vorderseite des zweiten Elektrolytlösungsaufbewahrungsteils (22), die der Haut oder der Schleimhautmembran (4) zugewandt ist;
- ein drittes Elektrolytlösungsaufbewahrungsteil (24) zum Aufbewahren einer Elektrolytlösung, wobei das dritte Elektrolytlösungsaufbewahrungsteil (24) an der Vorderseite der zweiten Kationen-Austausch-Membran (23) angeordnet ist, die der Haut oder der Schleimhautmembran (4) zugewandt ist; und
- eine zweite Anionen-Austausch-Membran (25), angeordnet an der Vorderseite des dritten Elektrolytlösungsaufbewahrungsteils (24), die der Haut oder der Schleimhautmembran (4) zugewandt ist.

## Revendications

1. Un dispositif d'iontophorèse (X1; X2) comportant une structure d'électrode active (1) composée :
d'une électrode (11) à laquelle une tension positive est appliquée;
d'un support de médicament (14) contenant une solution médicamenteuse contenant des ions de médicament chargés positivement, le support de médicament (14) étant placé sur la face avant de l'électrode faisant face à la peau ou à la membrane muqueuse (4)
**caractérisé en ce qu'**il comporte :
un film en résine à base de cellulose (15) placé sur la face avant du support de médicament (14) faisant face à la peau ou à la membrane muqueuse (4),
les ions de médicament étant administrés à travers le film en résine à base de cellulose (15).

2. Un dispositif d'iontophorèse (X1; X2) selon la Revendication 1, où le film en résine à base de cellulose (15) est placé sur la face avant d'une membrane d'échange de cations (17a) faisant face à la peau ou à la membrane muqueuse (4), les deux formant un film complexe (17) et
le film complexe (17) étant placé sur la face avant du support de médicament (14) faisant face à la peau ou à la membrane muqueuse (4),
les ions de médicament étant administrés à travers le film en résine à base de cellulose (15).

3. Un dispositif d'iontophorèse (X1; X2) selon la Revendication 2 où une interface de la membrane d'échange de cations (17a) et le film en résine à base de cellulose (15) sont liés, la membrane d'échange de cations (17a) étant intégrée avec le film en résine à base de cellulose (15).

4. Un dispositif d'iontophorèse (X1; X2) selon la Revendication 3 où :
une surface de la membrane d'échange de cations (17a) faisant face au film en résine à base de cellulose (15) est rugueuse ; et
les interfaces sont liées par une adhésion quelconque à l'aide d'un adhésif, d'une réaction de réticulation avec un agent de réticulation, ou d la formation du film en résine à base de cellulose (15) sur la membrane d'échange de cations (17a).

5. Un dispositif d'iontophorèse (X1; X2) selon l'une quelconque des Revendications 2 à 4 où la membrane d'échange de cations (17a) présente une structure dans laquelle un pore d'un film poreux est rempli d'une résine d'échange d'ions.

6. Un dispositif d'iontophorèse (X1; X2) selon l'une quelconque des Revendications 1 à 5 où un groupe d'échange de cation est introduit dans le film en résine à base de cellulose (15).

7. Un dispositif d'iontophorèse (X1; X2) selon l'une quelconque des Revendications 1 à 6 où le film en résine à base de cellulose (15) est rempli d'une résine d'échange d'ions avec un groupe d'échange de cations introduit dans ce dernier.

8. Un dispositif d'iontophorèse (X1; X2) selon l'une quelconque des Revendications 1 à7où:
la structure d'électrode active (1) comprend en outre un conteneur de solution électrolytique (12) destiné à maintenir une solution électrolytique en contact avec l'électrode (11), et une membrane d'échange d'anions (13) placée sur la face avant du conteneur de solution électrolytique (12) faisant face à la peau ou à la membrane muqueuse (4) ; et
le support de médicament (14) est placé sur la face avant de la membrane d'échange d'anions (13) faisant face à la peau ou à la membrane muqueuse (4).

9. Un dispositif d'iontophorèse (X1; X2) selon l'une quelconque des Revendications 1 à 8 comprenant en outre une contre-structure d'électrode (2) comprenant :
une deuxième électrode (21) à laquelle une tension négative est appliquée ;
un deuxième conteneur de solution électrolytique (22) destiné à maintenir une solution électrolytique en contact avec la deuxième électrode (21) ;
une deuxième membrane d'échange de cations (23) placée sur la face avant du deuxième conteneur de solution électrolytique (22) faisant face à la peau ou à la membrane muqueuse (4) ;
un troisième conteneur de solution électrolytique (24) contenant une solution électrolytique, le troisième conteneur de solution électrolytique (24) étant placé sur la face avant de la deuxième membrane d'échange de cations (23) faisant face à la peau ou à la membrane muqueuse (4) ; et
une seconde membrane d'échange d'anions (25) placée sur la face avant du troisième conteneur de solution électrolytique (24) faisant face à la peau ou à la membrane muqueuse (4).
